(19) Europäisches Patentamt
European Patent Office
Office européen des brevets

(11) **EP 3 416 696 B1**

(12) **FASCICULE DE BREVET EUROPEEN**

(45) Date de publication et mention de la délivrance du brevet:
**27.05.2020 Bulletin 2020/22**

(21) Numéro de dépôt: **17706450.8**

(22) Date de dépôt: **20.02.2017**

(51) Int Cl.:
***A61L 27/36*** *(2006.01)*

(86) Numéro de dépôt international:
**PCT/EP2017/053820**

(87) Numéro de publication internationale:
**WO 2017/140914 (24.08.2017 Gazette 2017/34)**

(54) **PROCEDE DE PREPARATION D'UN MATERIAU D'ALLOGREFFE, PRODUIT OBTENU, ET UTILISATIONS DE CELUI-CI**

VERFAHREN ZUR HERSTELLUNG EINES ALLOTRANSPLANTATIONSMATERIALS, ERHALTENES PRODUKT UND DESSEN VERWENDUNG

METHOD FOR PREPARING AN ALLOGRAFT MATERIAL, PRODUCT OBTAINED, AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats de validation désignés:
**MA**

(30) Priorité: **18.02.2016 FR 1651306**

(43) Date de publication de la demande:
**26.12.2018 Bulletin 2018/52**

(73) Titulaire: **TBF Genie Tissulaire (TBF)**
**69780 Mions (FR)**

(72) Inventeurs:
• **BARNOUIN, Laurence**
**38460 Vénérieu (FR)**
• **GUNZEL SAINT UPERY, Elise**
**31470 Fontenilles (FR)**

(74) Mandataire: **Tripoz, Inès**
**Cabinet Tripoz**
**Le Pôle Sud**
**22 rue Seguin**
**69002 Lyon (FR)**

(56) Documents cités:
**FR-A1- 2 949 042    US-A1- 2013 247 517**

• **DATABASE WPI Week 201502 1 avril 2013 (2013-04-01) Thomson Scientific, London, GB; AN 2015-006235 XP055328063, & CN 104 083 803 A (SHAANXI BAIAO REGENERATIVE MEDICINE CO) 8 octobre 2014 (2014-10-08)**
• **DEPAULA C A ET AL: "Effects of Hydrogen Peroxide Cleaning Procedures on Bone Graft Osteoinductivity and Mechanical Properties", CELL AND TISSUE BANKING, KLUWER ACADEMIC PUBLISHERS, DO, vol. 6, no. 4, 1 décembre 2005 (2005-12-01), pages 287-298, XP019234076, ISSN: 1573-6814, DOI: 10.1007/S10561-005-3148-2**

**Description**

La présente invention se rapporte, de manière générale, à la production de membranes issues de placenta de mammifères, et plus particulièrement à un procédé de préparation d'un matériau d'allogreffe formant une membrane viro-inactivée, lyophilisée et stérile issue de tissu placentaire de mammifère et constituée d'au moins une couche membranaire, ce matériau d'allogreffe étant adapté pour servir de greffon en chirurgie.

La membrane chorioamniotique, qui sépare le fœtus de l'endomètre de la mère chez les mammifères, inclut la membrane amniotique, ou amnios, et la membrane choriale, ou chorion. Ces deux membranes sont reliées par une membrane tissulaire spongieuse, aussi appelée couche spongieuse, constituée entre autres de collagène et de protéoglycanes, la membrane tissulaire spongieuse comportant des ponts protéiques, attachés de part et d'autre à l'amnios d'une part, et au chorion, d'autre part.

La membrane amniotique est la couche la plus interne de la membrane chorioamniotique. Elle a pour rôle de protéger le fœtus et de maintenir autour de lui le liquide amniotique. Cette membrane amniotique est un tissu très fin, transparent qui comporte plusieurs couches, à savoir une couche épithéliale, une membrane basale, une couche compacte et une couche fibroblastique.

La membrane choriale est la couche la plus externe de la membrane chorioamniotique, celle qui se trouve immédiatement en contact avec la paroi de l'utérus maternel (le decidua). Cette membrane choriale est un tissu épais et fibreux, comportant également plusieurs couches, à savoir une couche réticulaire, une membrane basale et une couche trophoblastique.

La membrane amniotique, obtenue à partir du placenta après un accouchement, est un tissu utilisé depuis plus de cent ans dans le traitement des brûlures et des blessures. En effet, dès 1910, Davies utilisait des membranes fœtales tant sur des brûlures que sur des tissus ulcérés. Trelford et Trelford-Sauder rapportent qu'en 1935 des auteurs ont publié des applications cliniques de la membrane amniotique en vaginoplastie, reconstruction conjonctivale, traitement des brûlures ou des blessures, et traitements relatifs à l'adhérence intra-abdominale. C'est en 1940 que De Roetth a utilisé pour la première fois une membrane fœtale en ophtalmologie reconstructive pour traiter des altérations conjonctivales de patients. Trelford *et al.* rapportent aussi qu'en 1952 Douglas a utilisé de l'amnios pour traiter des blessures étendues. Pour la première fois, il fut indiqué que la couche stromale de la membrane amniotique, comprenant la couche compacte et la couche fibroblastique, permettait une adhérence plus importante de la greffe, et donc de son efficacité. En 1972, les travaux de Trelford *et al.* ont confirmé ce fait. Gindraux *et al.* rapportent qu'à partir de 1972, et surtout depuis sa redécouverte en 1995, d'autres auteurs ont confirmé toutes les applications cliniques présentées précédemment, et ont également signalé de nouvelles indications telles que le tractus génito-urinaire, l'estomac, le larynx, la cavité orale, la tête et le cou, que ce soit dans des essais cliniques ou des rapports de cas.

Non vascularisée et non innervée, la membrane amniotique est riche en collagène et en divers facteurs de croissance, et présente des propriétés participant au processus de cicatrisation.

Avec les récents progrès de l'ingénierie tissulaire, il est apparu la nécessité d'utiliser des matrices servant de support destiné à l'adhérence et à la prolifération des cellules et des tissus, celles servant d'échafaudage à cette prolifération étant dérivées de tissus de mammifères. La membrane amniotique et la membrane choriale sont considérées comme une importante source de matrice support dans ce domaine.

Dans le cadre de l'activité de greffe tissulaire, la membrane amniotique peut servir de tissu de greffe qui peut être suturé ou collé avec une colle biologique. Le greffon ainsi implanté sert de matrice support qui est colonisée par les cellules du patient lors de la cicatrisation. Les utilisations les plus courantes de la membrane amniotique comme greffon en chirurgie sont, par exemple, la chirurgie ophtalmologique, et notamment les réparations de la cornée, la chirurgie gastrique pour les réparations d'ulcères, la chirurgie reconstructive de l'oreille, notamment les reconstructions du tympan, la chirurgie pour la réparation de lésions cutanées, notamment en réparation d'affections ulcéreuses, la chirurgie de réparation nerveuse, ligamentaire et tendineuse.

Un historique détaillé ainsi qu'une procédure de collecte, de conservation par congélation, et d'utilisation d'une membrane amniotique comme greffon en chirurgie ophtalmologique sont décrits dans le brevet US 6,152,142. Dans ce document, la technique décrite comprend une étape de retrait du chorion avant placement de l'amnios en solution de conservation avant congélation.

On connaît également l'utilisation de la membrane chorioamniotique complète comme greffe tissulaire, sans que l'amnios et le chorion n'aient été séparés. Les utilisations les plus courantes de la membrane complète chorioamniotique comme greffon en chirurgie sont, par exemple, dans la chirurgie ligamentaire, la chirurgie impliquant des fascia et les traitements de lésions et ulcères cutanées.

On connaît également des applications en injection intra-articulaire de membrane chorioamniotique broyée qui présenterait des propriétés liées à la limitation de l'arthrose.

Les membranes amniotiques cryoconservées ont également démontré leurs propriétés anti-inflammatoire, anti-fibrose et anti-angiogénique, de même que leur capacité à favoriser l'épithélialisation. Les membranes amniotiques traitées pour être décellularisées afin que ne demeurent que les matrices extracellulaires, sont donc des tissus reconnus pour leurs intérêts physiologiques dans le cadre de greffes tissulaires.

Cependant, il existe des problèmes de sécurité sanitaire ainsi que de conservation tissulaire pour ces produits dans une utilisation en tant que greffon.

Les membranes amniotiques, choriales, tissulaires spongieuses ou les membranes chorioamniotiques, étant des tissus biologiques, le risque de transmission d'une infection d'un donneur vers un receveur, ou de contamination lors de la collecte du tissu à l'accouchement, lors des traitements préparatoires du tissu, ou lors du transport de ce tissu est important.

On connait par exemple, du brevet US 8,709,494 des greffons obtenus par des procédés de préparation séparés de membrane amniotique et de membrane choriale, à partir d'une membrane chorioamniotique complète, avant de réassembler celles-ci par laminage avant déshydratation. On notera plus particulièrement que le procédé décrit dans ce document comprend une étape de séparation des membranes amniotique et choriale pour les nettoyer, la membrane retenue pour traitement étant, après nettoyage, chauffée dans une poche pour la déshydrater, à une température comprise entre 35°C et 50°C. Pour recueillir des greffons exempts de toute contamination pathogène, les greffons sont testés et sont récoltés et traités en milieu stérile ; le procédé décrit ne comprend aucune étape de désinfection. Cette sélection conduit à un taux de rejet des greffons très important.

De fait, un procédé efficace tant de désinfection que de conservation de ces tissus est vital pour la sécurité des patients.

La méthode de traitement par cryoconservation, bien qu'efficace, présente l'inconvénient de nécessiter l'installation de congélateurs à usage clinique qui sont coûteux et encombrants. Cette méthode implique également la contrainte de veiller à la conservation de la chaîne du froid entre le site de préparation des membranes amniotiques et le site d'utilisation, à savoir la salle d'opération chirurgicale ou au chevet du patient. De plus, l'utilisation de cryoconservateurs selon cette technique implique la nécessité de devoir les retirer des tissus avant utilisation. En effet, ces cryoconservateurs sont connus soit pour leur toxicité, par exemple le DMSO, soit pour les modifications structurelles qu'ils induisent sur les tissus, comme par exemple le glycérol.

Des méthodes alternatives ont été développées afin d'éviter cette contrainte technique par exemple des traitements qui comportent une étape de mise en présence des greffons avec des antibiotiques et ou des antimycotiques.

Par exemple, Nakamura proposa en 2004, dans Investigative Ophthalmology & Visual Science, January 2004, Vol. 45, No. 1, une méthode de désinfection, stérilisation et de lyophilisation de membrane amniotique à destination de la reconstruction d'une surface oculaire. L'étape de désinfection de la membrane amniotique est réalisée par incubation dans du tampon phosphate salin (PBS) contenant des antibiotiques et des antimycotiques. La décellularisation de la membrane amniotique est effectuée par incubation dans de l'acide éthylène diamine tétraacétique (EDTA). La membrane amniotique décellularisée est ensuite lyophilisée sous vide, le produit ainsi obtenu est conditionné et peut être conservé à température ambiante. Une dernière étape d'irradiation gamma à 25 kGy assure une stérilisation du produit final résultant.

Dans la demande de brevet CN 104 083 803 est décrit un procédé de traitement de l'amnios, comprenant notamment une première étape de désinfection effectuée en traitant la membrane avec un agent qui est une solution d'hydroxyde de sodium (0,1 à 0,5 M), de peroxyde d'hydrogène (1 à 3 g/L), de peroxyde d'acétate (0,1 à 0,5 g/L) ou d'éthanol (60 % à 90 %) ; suivie d'une deuxième étape en solution tampon comprenant un mélange d'antibiotiques. L'amnios est ensuite séché sous vide avant d'être conditionné.

L'utilisation d'antibiotiques pour la préparation de greffons destinés à être implantés présente de nombreux inconvénients notamment celui du risque d'induire des résistances ou d'utiliser des antiobiotiques dont le spectre n'est pas en cohérence avec les microorganismes présents dans les greffons traités.

Des méthodes de traitement sans antibiotiques sont également décrites sur des tissus mous ou des tissus plus résistants tels que l'os.

Dans la demande de brevet US 2013/0247517 est décrit un procédé de traitement d'un amnios destiné à servir de support de cicatrisation anti-adhésion dans le traitement des plaies. Le procédé décrit est relatif à un traitement chimique au glutaraldéhyde 1 %. Une décontamination du matériau est envisagée en trempant l'amnios dans une solution d'éthanol, ou de peroxyde d'hydrogène, ou les deux, sans plus de précision ni contrôle de la décontamination.

D'autres produits et procédés de traitement de tissus mous ont également été décrits dans la littérature brevets. Le brevet US 6,024,735 décrit, par exemple, des compositions et une méthode pour le nettoyage de tissus mous attachés à de l'os et obtenus à partir de cadavres. Les compositions en question comportent un détergent présentant des fonctions à base d'éther de lauryle et un autre détergent présentant des fonctions à base d'oxyéthylate d'alkyphénol, et sont commercialisées sous le nom d'Allowash™.

Dans le brevet US 6,482,584 est décrit un procédé de nettoyage par perfusion cyclique d'un implant poreux, pour l'inactivation de pathogènes et le produit nettoyé et stérilisé qui en résulte, par exemple des ligaments attachés à de l'os et destinés à former des allogreffes. Le procédé de traitement utilise, entre autres, le peroxyde d'hydrogène sous forme de solution aqueuse, et nécessite la mise en alternance de pressions positives et négatives au sein de la chambre de lavage dans laquelle est placé l'implant.

Dans la demande de brevet FR 2 949 042 au nom du demandeur est décrit un procédé de traitement de tissu de type cartilagineux destiné à servir de support en bioingénierie, ou servir de greffon. Ce procédé comprend notamment une étape de lavage à l'éthanol (70 à 100 %), une étape d'extraction des GAG, une étape de désinfection (dont notamment avec un alcool ou du peroxyde d'hydrogène (5 à 30 %), une étape de lyophilisation, et une étape de stérilisation en irradiation gamma.

Dans le document DePaula C.A. et al. « Effects of Hydrogen Peroxyde Cleaning Procédures on Bone Graft Osteoinductivity and Mechanical Properties », Cell Tissue Bank, 2005 ; 6(4):287-98, sont décrits les effets du nettoyage de tissus osseux par le peroxyde d'hydrogène sur les propriétés d'ostéo-induction et mécaniques des dits tissus. Le procédé comprend une première étape de traitement chimique au peroxyde d'hydrogène à 3 % et une seconde étape à l'alcool dénaturé à 70 %.

Les conditions de traitement appliquées sans antibiotiques ci-dessus citées sont adaptées à des tissus assez résistants tels que de l'os et même du ligament, mais appliquées à une couche membranaire issue de placenta pendant des temps et aux concentrations décrites comme présentant une efficacité suffisante pour garantir la décontamination, risqueraient de provoquer sa destruction pure et simple, ou tout au moins la perte de toutes les fonctionnalités biologiques et physiologiques d'intérêt, dont entre autres les différentes sous-couches, les facteurs de croissance, les composants de la matrice extracellulaire, tels que l'élastine, et la laminine.

En effet il est connu de Mbithi J. N. et al., Appl. Environ. Microbiol. 1990, vol 12, n°1, 147-179 que les virus non enveloppés comme le virus HAV n'est pas détruit par les solutions de peroxyde d'hydrogène à 3 ou 6 %.

De la même façon les abaissements des Poliovirus rapportés dans l'étude Reckitt et Colmann, 1997 : 1-12, sont limités à une diminution de 3 log malgré un traitement à des concentrations en $H_2O_2$ de 7,5 % pendant une durée de 6 heures.

De plus, la structure en couches et sous-couches des membranes amniotiques, choriales, tissulaires spongieuses ou les membranes chorioamniotiques nuit à l'efficacité des traitements sauf à détruire ladite structure au détriment de l'efficacité desdites membranes.

Un objectif de la présente invention est donc de proposer un procédé de préparation d'un matériau d'allogreffe formant une membrane viro-inactivée, lyophilisée et stérile issue de placenta de mammifère et constituée d'au moins une couche membranaire, le matériau d'allogreffe étant apte à servir de greffon en chirurgie, et selon lequel les conditions de traitement n'induisent pas de pertes ou de destruction d'au moins une couche membranaire ou des sous-couches la constituant, ou en tout cas, les minimisent.

Le procédé selon l'invention comprend au moins deux étapes de viro-inactivation et au moins une étape de lyophilisation selon lequel la première étape de viro-inactivation est effectuée en traitant ladite au moins une couche membranaire avec un premier agent de viro-inactivation choisi dans la famille des alcools ; et une deuxième étape de viro-inactivation est effectuée après ladite première étape de viro-inactivation, en traitant ladite au moins une couche membranaire avec un deuxième agent de viro-inactivation choisi dans la famille des peroxydes.

Un autre objectif de la présente invention est aussi de proposer un procédé de préparation d'un matériau injectable issu de la membrane tissulaire spongieuse viro-inactivée, lyophilisée et stérile issue de placenta de mammifère.

Le produit obtenu selon le procédé est également décellularisé, stérilisé et conditionné tout en conservant ses propriétés d'intérêt chirurgical pour remplir sa fonction de greffon ou de matériau injectable. Les avantages techniques de ce procédé par rapport aux procédés existants sont :

- d'assurer un traitement chimique viro-inactivant et désinfectant du tissu en profondeur pour assurer la sécurité du greffon ou du matériau injectable. Ce traitement permet ainsi de ne pas avoir à séparer la membrane amniotique de la membrane choriale, conservant de fait la structure chorioamniotique et les ponts protéiques, par exemple de collagène, liant ces deux membranes ;

- de fournir un traitement chimique désinfectant du tissu de telle sorte que l'obligation d'asepsie des tissus ou l'utilisation empirique d'antibiotiques ne sont pas requises comme dans le cas d'un procédé par cryoconservation, ce traitement chimique présentant aussi l'avantage technique de pouvoir utiliser des tissus placentaires obtenus par accouchement par voie basse, pourtant réputés comme ayant connu une contamination microbienne selon cette voie,

- de fournir une méthode simple de lyophilisation telle que la membrane amniotique, ou choriale, ou chorioamniotique après traitement selon le procédé se présente comme parfaitement plate, à savoir sans enroulement de la membrane sur elle-même, et sans présenter un aspect gondolé. En effet, les procédés de préparation connus de l'art antérieur mettant en œuvre un traitement chimique suivi d'une étape de lyophilisation ont tendance à faire gondoler la membrane sur ses bords pendant cette étape.

Définitions utilisées dans la présente demande.

Certaines expressions ou mots peuvent être utilisés dans la présente demande pour indiquer des éléments techniques, faits, propriétés ou caractéristiques. Leur signification est donnée ci-après :

- par « couche membranaire », on entend l'un des feuillets tissulaires constitutifs du placenta. Dans la présente invention il s'agit soit de la membrane amniotique, soit de la membrane choriale, soit de la membrane tissulaire spongieuse, soit de la membrane chorioamniotique, chacun de ces feuillets pouvant inclure, ou non, les sous-structures physiologiques constitutives de l'amnios et du chorion.
- par « membrane amniotique », ou « amnios », on entend l'enveloppe tissulaire qui se développe autour de l'embryon, puis du foetus, chez le mammifère durant la grossesse. Elle a pour rôle de protéger l'organisme en développement en maintenant autour de lui le liquide amniotique. Elle s'accole à la deuxième membrane qui est le chorion. La membrane amniotique inclut les sous-couches physiologiques suivantes : la couche cellulaire épithéliale, la membrane basale, la couche compacte, la couche fibroblastique, la couche spongieuse.
- par « membrane choriale », ou « chorion », on entend l'enveloppe tissulaire située entre l'amnios et la paroi utérine qui se développe autour de l'embryon, puis du foetus, chez le mammifère durant la grossesse. La membrane choriale inclut les sous-couches physiologiques suivantes : la couche réticulaire, la membrane basale, la couche cellulaire trophoblastique.
- par « membrane chorioamniotique », on entend l'ensemble amnios et chorion, qui n'ont pas été séparés naturellement ou artificiellement, ceux-ci étant reliés par des ponts protéiques.
- par « membrane tissulaire spongieuse », ou « couche spongieuse », on entend une couche située entre l'amnios et le chorion.
- par « viro-inactivation », on entend une technique qui permet de réduire fortement ou totalement, et définitivement, la capacité d'action des virus. Ceux-ci, définis comme inactivés, perdent leurs capacités pathogéniques et de réplication par une diminution de leur population de 4 log lors des titrages résiduels qui suivent une ou deux étapes chimiques indépendantes, que ce soit sur des virus enveloppés, ou non enveloppés, ADN ou ARN.
- par « lyophilisation », on entend une technique visant à dessécher un produit préalablement surgelé par sublimation. Plus précisément, le liquide à ôter du produit est dans un premier temps transformé en glace par congélation ; puis par une dessiccation primaire, sous vide, la glace est sublimée ; enfin par une dessiccation secondaire, les molécules d'eau à la surface du produit sont extraites par désorption.
- par « ne pas endommager l'intégrité structurelle, fonctionnelle et biologique de la couche membranaire », on entend que le produit obtenu selon le procédé présente une conservation suffisante des molécules biologiques assurant la structure mécanique du tissu, tels que le collagène de divers types, les protéoglycanes, l'élastine, et la laminine, ainsi que des molécules biologiquement actives, comme par exemple les hormones, enzymes, facteurs de croissance, afin d'assurer une utilisation ultérieure du produit. Ainsi, selon ces paramètres, le produit obtenu selon le procédé présente des propriétés biologiques similaires ou quasi-similaires au tissu initial avant traitement. Dans la présente invention, ces molécules sont par exemple la laminine et l'élastine ; ainsi que les facteurs de croissance de type EGF (Epidermal Growth Factor), TGF (Transforming Growth Factor) et KGF (Keratinocyte Growth Factor).
- par « alcools », on entend tout composé organique dont l'un des carbones est lié à un groupe hydroxyle (-OH).
- par « peroxydes », on entend tout composé chimique contenant un groupe fonctionnel de formule générale R-O-O-R' (deux atomes d'oxygène voisins liés entre eux) où R et R' sont des atomes d'hydrogène ou des radicaux alkyls quelconques.

Le tissu physiologique destiné à être traité selon le procédé décrit dans la présente invention est une membrane amniotique, une membrane choriale, une membrane tissulaire spongieuse ou une membrane chorioamniotique encore associée au placenta. Celle-ci est obtenue après l'accouchement d'un donneur proprement informé et consentant en accord avec exigences des directives Européennes et la Loi Bioéthique. Cet accouchement peut s'être déroulé par césarienne ou par voie basse. Par exigence sanitaire relative aux dons de tissus et de cellules d'origine humaine, une qualification socio-clinique du donneur et biologique en amont du donneur est systématique. Cette qualification implique une recherche de virus HIV, hépatites B, C, HTLV et de la bactérie tréponème pâle responsable de la syphilis. Le tissu placentaire est avantageusement placé dans une boîte stérile, congelé et transporté à une température de -20°C, puis stocké à -80°C. Le tissu placentaire peut être aussi, lors du prélèvement, transporté à +4 °C pour être préparé. Si les données cliniques du donneur sont conformes aux exigences relatives au don tissulaire, et que l'analyse sanguine de recherche de bactéries et virus se révèle négative, le tissu éligible peut subir le procédé de préparation éventuellement après décongélation.

Le tissu physiologique d'intérêt est séparé du placenta, un nettoyage et un contrôle qualité sont effectués. Le tissu physiologique peut être une membrane amniotique naturellement séparée de la membrane choriale pendant l'accouchement, ou artificiellement séparée par une intervention mécanique humaine ; ou une membrane choriale naturellement séparée de la membrane amniotique pendant l'accouchement, ou artificiellement séparée par une intervention humaine ; ou encore une membrane chorioamniotique entière, sans que la séparation entre la membrane amniotique et la membrane choriale n'ait été opérée. La membrane tissulaire spongieuse, naturellement présente entre la membrane amniotique et la membrane choriale peut être retirée ou non retirée.

Si le tissu placentaire n'a pas été congelé initialement, la découpe et la séparation des différentes membranes du placenta peuvent être réalisées après le transport à +4°C, les membranes obtenues peuvent être conservées à -80°C.

Le placenta non séparé de ses tissus physiologiques peut être congelé pour conservation à une température de -80°C pendant une durée allant jusqu'à 2 ans si le tissu physiologique n'a pas à être immédiatement traité.

Le procédé selon l'invention est caractérisé par une succession d'étapes non interchangeables et appliquées au tissu physiologique qui est une couche membranaire qui peut être préalablement découpée ou non le cas échéant.

Selon un autre mode de réalisation, la membrane tissulaire spongieuse est extraite par pression de la membrane amniotique pour ne conserver que la couche d'intérêt. Alternativement, cette membrane tissulaire spongieuse peut également être collectée et conservée dans un flacon sous vide.

Afin de garantir l'innocuité du produit fini en tant que matériau d'allogreffe, une première phase de traitement chimique est effectuée. En effet, le produit fini doit être exempt d'agents microbiologiques tels que bactéries, virus, parasites.

La couche membranaire à l'état congelé peut être avantageusement lavée dans de l'eau purifiée ou séjourne dans un bain d'eau purifiée. Cette étape assure tant la décongélation du tissu si nécessaire jusqu'à la température ambiante, qu'une lyse des cellules présentes dans le tissu par effet de pression osmotique.

La première étape de traitement chimique viro-inactivant est l'application à la couche membranaire d'un lavage, ou le séjour de cette dernière dans un bain, composé d'un premier agent viro-inactivant qui est de l'éthanol. Un lavage de la couche membranaire par de l'eau purifiée ou un séjour dans un bain d'eau purifiée peut être avantageusement effectué après cette étape.

Selon un mode de réalisation, le premier agent de viro-inactivation est l'éthanol à une teneur en alcool comprise entre 50% et 80%, et de préférence à 70% v/v.

Selon un autre mode de réalisation, la première étape de viro-inactivation est effectuée en traitant la couche membranaire avec de l'éthanol à 70% v/v pendant environ 60 minutes.

La deuxième étape du traitement chimique viro-inactivant est l'application à la couche membranaire d'un lavage, ou le séjour de cette dernière dans un bain, composé d'un deuxième agent de viro-inactivation qui est le peroxyde d'hydrogène.

Comme exposé ci-dessus, il est connu que le traitement par des solutions de peroxyde d'hydrogène n'est efficace sur les virus sans enveloppés qu'à des concentrations supérieures à 10 %.

Il a par ailleurs été mis en évidence par la demanderesse que le traitement des membranes par des solutions de peroxyde d'hydrogène à des concentrations supérieures à 10 % pendant des durées nécessaires à l'inactivation de virus non enveloppés détruit lesdites membranes.

Le deuxième agent de viro-inactivation est le peroxyde d'hydrogène sous une forme choisie parmi une solution aqueuse, et un gaz.

Selon un mode de réalisation, le deuxième agent de viro-inactivation est le peroxyde d'hydrogène sous forme de solution aqueuse dans une concentration comprise entre 3% et 30% w/v.

De façon surprenante il a été mis en évidence que scinder la deuxième étape de viro-inactivation de ladite au moins une couche membranaire en deux sous-étapes de traitement par le peroxyde d'hydrogène à différentes concentrations et pendant des durées différentes permettait d'obtenir une efficacité inattendue sur notamment les virus non enveloppés.

Le procédé est caractérisé en ce que la deuxième étape de viro-inactivation de ladite au moins une couche membranaire comporte deux sous-étapes de traitement par le deuxième agent de viro-inactivation choisi dans la famille des peroxydes.

Procédé de préparation d'un matériau d'allogreffe formant une membrane viro-inactivée, lyophilisée et stérile issue de placenta de mammifère et constituée d'au moins une couche membranaire, ledit procédé comprenant au moins deux étapes de viro-inactivation et au moins une étape de lyophilisation et selon lequel :

- la première étape de viro-inactivation est effectuée en traitant ladite au moins une couche membranaire avec un premier agent de viro-inactivation choisi dans la famille des alcools ;
- une deuxième étape de viro-inactivation est effectuée après ladite première étape de viro-inactivation, en traitant ladite au moins une couche membranaire avec un deuxième agent de viro-inactivation choisi dans la famille des peroxydes, caractérisé en ce que la deuxième étape de viro-inactivation de ladite au moins une couche membranaire comporte deux sous-étapes de traitement par le deuxième agent de viro-inactivation choisi dans la famille des peroxydes.

La deuxième étape de viro-inactivation de ladite au moins une couche membranaire comporte deux sous-étapes de traitement par le peroxyde d'hydrogène :

- une première sous-étape de traitement s'effectuant avec une solution de peroxyde d'hydrogène à un concentration supérieure à 10% w/v pendant moins de 20 minutes ; et

- une deuxième sous-étape de traitement s'effectuant avec une solution de peroxyde d'hydrogène à un concentration inférieure à 5 % w/v pendant une durée supérieure à 50 minutes.

Selon un autre mode de réalisation, la deuxième étape de viro-inactivation de ladite au moins une couche membranaire comporte deux sous-étapes de traitement par le peroxyde d'hydrogène :

- une première sous-étape de traitement s'effectuant avec une solution de peroxyde d'hydrogène à 30% w/v pendant environ 15 minutes ; et
- une deuxième sous-étape de traitement s'effectuant avec une solution de peroxyde d'hydrogène à 3% w/v pendant environ 60 minutes.

A la fin de cette deuxième étape de viro-inactivation, on procède avantageusement à une étape de neutralisation, par application à la couche membranaire d'un lavage, ou le séjour de cette dernière dans un bain, composé d'au moins un tampon basique.

Selon un mode préféré de réalisation, deux bains sont effectués dans un tampon basique contenant de l'hydroxyde de sodium dilué et dont le pH est ajusté à 8,5.

L'étape suivante de traitement chimique de la couche membranaire est avantageusement l'application à la couche membranaire d'un lavage, ou le séjour de cette dernière dans un bain, composé d'au moins une solution tampon assurant un rééquilibrage physiologique de la couche membranaire.

Selon un mode préféré de réalisation, deux bains sont effectués en solution de PBS.

Selon un mode préféré de réalisation, les étapes de décongélation, viro-inactivation, lavage, ajustage de pH et mise en tampon s'effectuent à température ambiante.

Suite à cette première phase de traitement chimique, la couche membranaire subit une seconde phase de traitement qui est une lyophilisation. Cette seconde phase permet à la fois d'assurer la qualité de conservation sous vide du produit obtenu selon le procédé, et d'assurer une étape supplémentaire de désinfection et de décellularisation à la couche membranaire suite au traitement chimique.

Selon un mode préféré de réalisation, le procédé de lyophilisation de la couche membranaire est réalisé entre deux supports sensiblement plats.

Selon un mode davantage préféré de réalisation, le procédé de lyophilisation de la couche membranaire est réalisé par prise en sandwich de la couche membranaire entre deux supports sensiblement plats, perméables au gaz, dont la vapeur d'eau, et dont la surface en appui de la couche membranaire est sensiblement hydrophobe empêchant l'adhérence connue des produits de cellulose à des résidus cellulaires de la couche épithéliale de l'amnios.

Selon un mode encore davantage préféré de réalisation, le procédé de lyophilisation de la couche membranaire est réalisé par prise en sandwich de la couche membranaire entre deux supports sensiblement plats en papier de cellulose ou dérivé de cellulose.

La couche membranaire viro-inactivée est donc soumise à une lyophilisation dans les conditions suivantes :

- une congélation en deux étapes :

  - la première étape de congélation s'effectuant à une température d'acclimatation choisie pour ne pas endommager l'intégrité structurelle, fonctionnelle et biologique de la couche membranaire,
  - la deuxième étape de congélation s'effectuant à la température finale de congélation qui est inférieure à la température d'acclimatation ;

- une lyophilisation en deux étapes principales, dites primaire et secondaire :

  - l'étape de lyophilisation primaire s'effectuant par application d'un vide à environ 200 microbars et d'un profil de température ascendant ;
  - l'étape secondaire de lyophilisation s'effectuant par application d'un vide à environ 50 microbars et d'un profil de température descendant.

Dans un mode de réalisation la température d'acclimatation est comprise entre - 5 et - 20°C et la température finale de congélation est comprise entre - 40 et - 60 °C.

Le profil de température ascendant est avantageusement un profil selon lequel la température de lyophilisation est initialement réglée à une température initiale basse puis augmentée vers une température finale de lyophilisation primaire, en une ou plusieurs étapes de température ascendantes intermédiaires. Le profil de température descendant est avantageusement un profil selon lequel la température de lyophilisation est initialement réglée à une température supérieure à la température finale de l'étape de lyophilisation primaire, puis qui est ensuite descendue vers une température finale de lyophilisation secondaire supérieure à la température initiale de l'étape de lyophilisation primaire.

Ces conditions de lyophilisation permettent d'obtenir un produit, qui est une couche membranaire viro-inactivée et lyophilisée, qui est notamment caractérisé en ce qu'il présente une teneur en eau résiduelle à température ambiante inférieure à 5%.

Le produit obtenu, qui est une couche membranaire viro-inactivée et lyophilisée, se présente comme parfaitement à plat. Il peut être avantageusement découpé selon des paramètres préalablement définis afin de correspondre aux besoins du praticien réalisant la greffe du produit obtenu chez un patient.

Le produit obtenu, qui est une couche membranaire viro-inactivée et lyophilisée, se présente comme parfaitement à plat et est conditionnable sous cette forme dans un emballage stérile sous conditions stériles.

Selon un autre mode de réalisation, le produit obtenu est une membrane tissulaire spongieuse viro-inactivée et lyophilisée.

La couche membranaire viro-inactivée et lyophilisée, et qui est emballée, subit une dernière étape de stérilisation qui est une irradiation gamma.

Selon un mode préféré de réalisation, cette étape de stérilisation s'effectue par exposition de la couche membranaire à des rayonnements gamma à 25-32 kGrays.

Le produit obtenu selon le procédé est donc un matériau d'allogreffe viro-inactivé, lyophilisé et stérile, constitué d'au moins une couche membranaire choisie parmi la membrane amniotique, la membrane choriale, et la membrane chorioamniotique dans laquelle l'amnios et le chorion sont reliés entre eux par des ponts protéiques.

Le produit obtenu selon le procédé peut être aussi un matériau injectable viro-inactivé, lyophilisé et stérile, constitué d'au moins une couche membranaire qui est la membrane tissulaire spongieuse.

Le produit obtenu selon le procédé est utilisable comme matériau d'allogreffe choisi dans le groupe consistant en la greffe de ligament, la greffe de fascia lata, la greffe de tendon, la greffe de dure-mère, la greffe de membrane sous-muqueuse, la greffe de manchon nerveux, la greffe ligamentaire, la greffe de cartilage la greffe de coiffe rotateur, la greffe de tissu conjonctival, les agents de traitement de plaies et/ou d'ulcères, et les agents d'amélioration de sutures.

Quelques utilisations envisageables du produit obtenu selon le procédé sont, entre autres et notamment, la réparation d'ulcères de cornée, la réparation de tendon et/ou de ligaments, le remplacement ligamentaire, la réparation nerveuse par manchonnage, la réparation cartilagineuse, le remplacement de la dure-mère, le remplacement de membrane sous-muqueuse, le renforcement de la coiffe rotateur, le traitement des ulcères conjonctivaux et/ou cornéens, le traitement des ulcères et/ou des plaies cutanées, et l'amélioration des cicatrisations et des sutures.

Quelques utilisations envisageables du produit aussi obtenu selon le procédé à partir de la membrane tissulaire spongieuse sont par exemple le traitement d'injection intra-articulaire de l'état préarthrosique ou arthrosique et la réparation ligamentaire, méniscale ou cartilagineuse.

Une autre utilisation envisageable du produit selon l'invention est par exemple une matrice support d'ingénierie tissulaire pour culture de cellules mésenchymateuses ou fibroblastes.

L'invention sera mieux comprise et complétée en se référant utilement aux figures, incluses à titre purement illustratif, et dont le résumé est donné ci-après :

Brève Description des Figures.

Figure 1 : Représentation synthétique des étapes du procédé de préparation du matériau d'allogreffe.

Figure 2a : Photographie en microscopie optique d'une coupe d'une membrane chorioamniotique non traitée, colorée à l'Hématoxyline-Eosine. Le grossissement employé est de x40. (1) désigne la membrane amniotique. (2) désigne la membrane tissulaire spongieuse. (3) désigne le chorion.

Figure 2b : Photographie en microscopie optique d'une coupe d'une membrane chorioamniotique traitée selon le procédé, colorée à l'Hématoxyline-Eosine. Le grossissement employé est de x40. (1) désigne la membrane amniotique. (2) désigne la membrane tissulaire spongieuse. (3) désigne le chorion.

Il sera fait référence aux figures ainsi identifiées en tant que de besoin dans la description détaillée ci-après d'un exemple d'un mode de réalisation de l'invention.

**Exemple 1 : Exemple d'un mode de réalisation de l'invention où une couche membranaire est traitée selon le procédé afin de devenir un matériau d'allogreffe.**

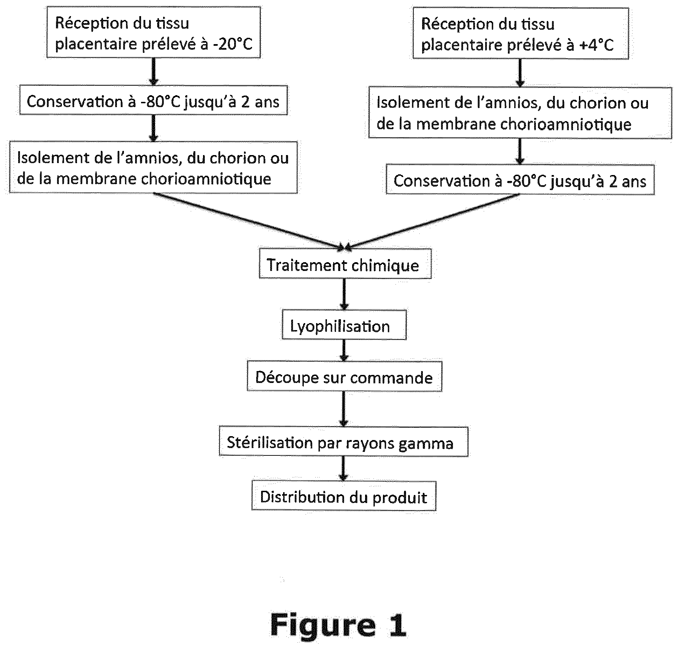

On se référera utilement à la Figure 1 pour visualiser les étapes importantes du procédé.

Un donneur proprement informé et consentent en accord avec les exigences de la Déclaration d'Helsinki offre en don le tissu placentaire issu d'un accouchement. Cet accouchement peut s'être déroulé par césarienne ou par voie basse. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le tissu placentaire est récupéré le plus tôt possible en salle d'accouchement suite à un accouchement, que ce soit par voie basse ou par césarienne. Il peut être avantageusement placé dans une boîte stérile puis congelé à une température de -20°C.

Ce tissu placentaire isolé peut être conservé à sec à une température de - 80°C jusqu'à une durée de deux ans ou être directement traité selon le procédé.

Au laboratoire, en salle stérile, la procédure suivante est appliquée :

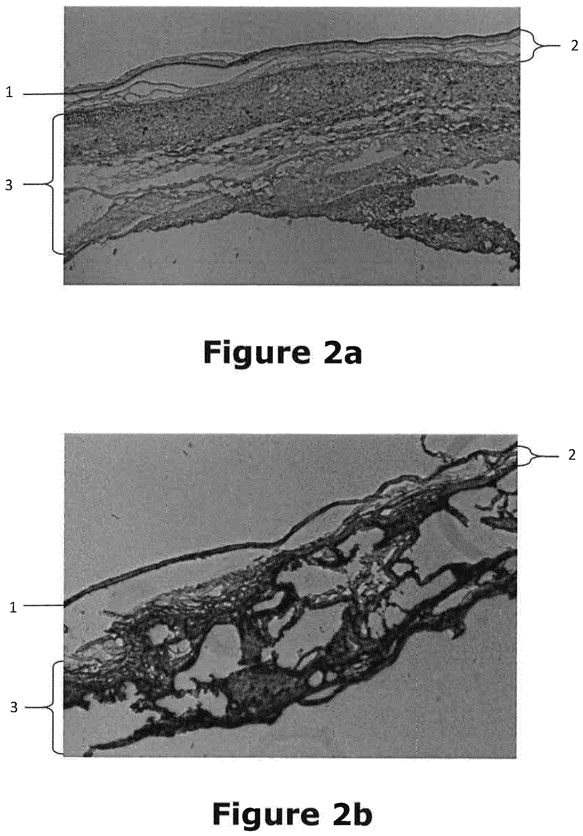

La couche membranaire d'intérêt, qui peut être par exemple l'amnios, le chorion, ou la membrane chorioamniotique, est isolée du placenta et est nettoyée. Une photographie en microscopie optique d'une membrane chorioamniotique non encore traitée et colorée à l'Hématoxyline-Eosine est présentée à la Figure 2a, les différentes couches la composant sont indiquées, à savoir : (1) désigne la membrane amniotique. (2) désigne la membrane tissulaire spongieuse. (3) désigne le chorion.

Alternativement, après prélèvement, le tissu placentaire peut être placé à environ +4°C. En salle stérile, la couche membranaire d'intérêt est isolée et est nettoyée. Cette couche membranaire peut être conservée à sec à une température de -80°C jusqu'à une durée de deux ans.

La couche membranaire subit une succession de bains assurant un traitement chimique de celle-ci. Ce traitement a pour objectif une désinfection de la couche membranaire d'intérêt, et tout particulièrement sa viro-inactivation. Une agitation douce à environ 30 rpm du milieu liquide est appliquée lors de chaque bain afin d'assurer une pénétration homogène des solvants dans les tissus, tout en évitant de rompre les ponts protéiques entre le chorion et l'amnios si la couche membranaire sur laquelle est appliquée le procédé est la membrane chorioamniotique.

Dans un premier temps, la couche membranaire est déposée dans un bain d'eau purifiée à température ambiante pendant environ 3 heures. Cette étape assure tant la décongélation du tissu physiologique qu'une première étape de lyse cellulaire par pression osmotique.

Puis, la couche membranaire est transférée dans un bain décontaminant composé d'éthanol 70% v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, la couche membranaire est transférée dans un bain composé de peroxyde d'hydrogène à 30% w/v à température ambiante pendant environ 15 minutes.

Puis la couche membranaire est transférée dans un bain décontaminant composé de peroxyde d'hydrogène à 3% w/v à température ambiante pendant environ 1 heure.

L'action chimique appliquée à la couche membranaire peut ensuite être neutralisée dans au moins un bain comportant, par exemple, de l'hydroxyde de sodium dilué autour d'un pH de 8,5. Le au moins un bain de neutralisation s'effectue à température ambiante pendant environ 15 minutes. On a trouvé des résultats intéressants à cette étape de neutralisation en utilisant deux bains à l'hydroxyde de sodium dilué à pH 8,5.

Afin d'assurer un rééquilibrage du pH et pour éliminer au mieux les résidus organiques se détachant du tissu d'intérêt, la couche membranaire est transférée dans au moins un bain de tampon physiologique, choisi de façon appropriée, afin d'assurer son rééquilibrage physiologique. Le au moins un bain s'effectue à température ambiante pendant environ 15 minutes. On a trouvé des résultats intéressants à cette étape en utilisant deux bains successifs en solution de PBS.

Enfin, la couche membranaire peut être transférée dans un dernier bain à l'eau purifiée, à température ambiante, pendant au moins 15 minutes et jusqu'à environ 1 heure.

A cette étape du procédé, on peut considérer que la couche membranaire obtenue selon ce traitement chimique peut être définie comme une membrane en grande partie désinfectée notamment viro-inactivée, et décellularisée.

Suite à cette partie relative aux traitements chimiques, la couche membranaire subit un traitement de lyophilisation.

Sur un plateau en inox, la couche membranaire est avantageusement placée entre deux couches de filtres support, de préférence en cellulose ou dérivé de cellulose comme par exemple de la méthylcellulose maillée pour faciliter les échanges de vapeur d'eau.

Si la couche membranaire est définie comme étant l'amnios, qui est un tissu particulièrement fin, afin d'éviter tout risque de repliement de la couche membranaire pendant l'étape de lyophilisation, une grille est disposée au-dessus de celle-ci, qui est prise en sandwich entre les deux filtres supports, tel qu'environ 1 mm sépare la grille du filtre support supérieur.

L'ensemble précédemment décrit est transféré dans un lyophilisateur où une étape de congélation suivie d'une étape de lyophilisation sont effectuées selon les modalités suivantes :

- Congélation :

    - La première étape de congélation s'effectue à une température d'acclimatation choisie pour ne pas endommager l'intégrité structurelle, fonctionnelle et biologique de la couche membranaire ;
    - la deuxième étape de congélation s'effectue à la température finale de congélation qui est inférieure à la température d'acclimatation ;

- Lyophilisation :

    - Une étape de lyophilisation primaire s'effectue par application d'un vide à environ 200 microbars et par application d'un profil de température ascendant ;
    - Une étape secondaire de lyophilisation s'effectue par application d'un vide à environ 50 microbars et par application d'un profil de température descendant.

Suite à cette étape de lyophilisation, la couche membranaire selon ce traitement peut être définie comme une membrane désinfectée, notamment viro-inactivée, décellularisée et lyophilisée. La couche membranaire ne présente ni bords retournés, ni un aspect gondolé ou gaufré, et se présente comme parfaitement à plat. Une photographie par microscopie optique d'une coupe de membrane chorioamniotique traitée selon le procédé est présentée à la Figure 2b, les différentes couches la composant sont indiquées, à savoir : (1) désigne la membrane amniotique. (2) désigne la membrane tissulaire spongieuse. (3) désigne le chorion.

La couche membranaire obtenue peut être avantageusement découpée à cette étape du procédé. Les professionnels de santé, pour lesquels le produit final est destiné, peuvent fournir les paramètres de découpage exacts selon leurs besoins d'exercice.

La membrane viro-inactivée et lyophilisée obtenue est conditionnée à plat en conditions stériles.

L'extraction des membranes, qui est la membrane tissulaire spongieuse, peut être conditionnée en flacon sous vide.

En dehors de la salle stérile, une dernière étape de stérilisation de la couche membranaire viro-inactivée et lyophilisée est effectuée par exposition de celle-ci à des rayonnements gamma à 25-32 kGrays.

Le produit ainsi obtenu peut être distribué pour être utilisé comme matériau d'allogreffe, par exemple, la greffe de ligament, la greffe de fascia lata, la greffe de tendon, la greffe de dure-mère, la greffe de membrane sous-muqueuse, la greffe de manchon nerveux, la greffe de cartilage, la greffe de coiffe rotateur, la greffe de tissu conjonctival, les agents de traitement de plaies et/ou d'ulcères, et les agents d'amélioration de cicatrisation et des sutures.

Quelques utilisations envisageables du produit obtenu selon le procédé sont donc, entre autres et notamment comme indiquées ci-dessus, la réparation d'ulcères de cornée, la réparation de tendon et/ou de ligament, le remplacement ligamentaire, la réparation nerveuse par manchonnage, le remplacement de la dure-mère, le remplacement de membrane sous-muqueuse, le renforcement de la coiffe des rotateurs, le traitement des ulcères conjonctivaux et/ou cornéens, le traitement des ulcères et/ou des plaies cutanées, et l'amélioration des sutures.

**Exemple 1 bis : Exemple d'un mode de réalisation de l'invention où une couche membranaire est traitée selon le procédé afin de devenir un matériau d'allogreffe.**

On se référera utilement à la Figure 1 pour visualiser les étapes importantes du procédé.

Un donneur proprement informé et consentant en accord avec les exigences de la Déclaration d'Helsinki offre en don le tissu placentaire issu d'un accouchement qui s'est déroulé par voie basse. Le test de dépistage du donneur pour les virus HIV, hépatites B, C, HTLV et de la bactérie tréponème pâle responsable de la syphilis est négatif.

Le tissu placentaire est récupéré le plus tôt possible en salle d'accouchement suite à l'accouchement. Il est placé dans une boîte stérile puis congelé à une température de -20°C et transporté au laboratoire.

Au laboratoire, en salle stérile, la procédure suivante est appliquée :

La couche membranaire d'intérêt, qui est la membrane chorioamniotique, est isolée du placenta et est nettoyée.

La couche membranaire subit une succession de bains assurant un traitement chimique de celle-ci. Une agitation douce à 30 rpm du milieu liquide est appliquée lors de chaque bain afin d'assurer une pénétration homogène des solvants dans les tissus, tout en évitant de rompre les ponts protéiques entre le chorion et l'amnios de la membrane chorioamniotique.

Dans un premier temps, la couche membranaire est déposée dans un bain d'eau purifiée à température ambiante pendant 3 heures. Cette étape assure tant la décongélation du tissu physiologique qu'une première étape de lyse cellulaire par pression osmotique.

Puis, la couche membranaire est transférée dans un bain décontaminant composé d'éthanol 70% v/v à température ambiante pendant 1 heure.

Un lavage est effectué dans de l'eau purifiée pendant 15 minutes à température ambiante pour retirer l'éthanol.

Afin d'assurer la seconde étape de traitement décontaminant, la couche membranaire est transférée dans un bain composé de peroxyde d'hydrogène à 30% w/v à température ambiante pendant 15 minutes.

Puis la couche membranaire est transférée dans un bain décontaminant composé de peroxyde d'hydrogène à 3% w/v à température ambiante pendant 1 heure.

L'action chimique appliquée à la couche membranaire est neutralisée dans deux bains successifs d'hydroxyde de sodium dilué à un pH de 8,5. Chacun des bains de neutralisation s'effectue à température ambiante pendant 15 minutes.

Afin d'assurer un rééquilibrage du pH et pour éliminer au mieux les résidus organiques se détachant du tissu d'intérêt, la couche membranaire est transférée dans deux bains successifs en solution de PBS afin d'assurer son rééquilibrage physiologique. Chacun des bains s'effectue à température ambiante pendant 15 minutes.

Enfin, la couche membranaire est transférée dans un dernier bain à l'eau purifiée, à température ambiante, pendant 15 minutes.

Suite à cette partie relative aux traitements chimiques, la couche membranaire subit un traitement de lyophilisation.

Sur un plateau en inox, la couche membranaire est placée entre deux couches de filtres support en méthylcellulose maillée pour faciliter les échanges de vapeur d'eau.

L'ensemble précédemment décrit est transféré dans un lyophilisateur où une étape de congélation suivie d'une étape de lyophilisation sont effectuées selon les modalités suivantes :

Congélation :

La première étape de congélation s'effectue à une température de -10°C pendant 5 minutes, puis à -15°C pendant 90 minutes ;

la deuxième étape de congélation s'effectue à une température de -50°C pendant 125 minutes ;

Lyophilisation :

Une étape de lyophilisation primaire s'effectue par application d'un vide à 200 microbars et par application d'une température de +10°C pendant 8 heures, suivie par une température de +25°C pendant 150 minutes ;

Une étape secondaire de lyophilisation s'effectue par application d'un vide à 50 microbars et par application d'une température de +35°C pendant 5 heures, suivie par une température de +25°C pendant 1 heure.

Suite à cette étape de lyophilisation, la couche membranaire ne présente ni bords retournés, ni un aspect gondolé ou gaufré, et se présente comme parfaitement à plat.

La couche membranaire obtenue est découpée à cette étape du procédé en rectangles de 20 sur 10 cm (ou 20 sur 20 cm) rendant celle-ci apte à être utilisée comme matériau d'allogreffe dans la réparation du ligament.

La membrane viro-inactivée et lyophilisée obtenue est conditionnée à plat en conditions stériles.

En dehors de la salle stérile, une dernière étape de stérilisation de la couche membranaire viro-inactivée et lyophilisée est effectuée par exposition de celle-ci à des rayonnements gamma à 25-32 kGrays.

Le produit ainsi obtenu est distribué pour être utilisé comme matériau d'allogreffe pour une utilisation dans la réparation du ligament.

**Exemple 2 : Démonstration de l'efficacité de la viro-inactivation de la couche membranaire selon le procédé.**

Des mesures de la charge virale ont été effectuées dans des membranes chorioamniotiques, par injection de virus entre l'amnios et le chorion afin de vérifier la viro-inactivation des tissus selon le procédé.

Des aliquotes des membranes ainsi traitées sont prélevées afin d'être mises en solution et centrifugées. La fraction soluble sert à ensemencer des cellules en milieu de culture cellulaire. Chaque type de virus étudié est ensemencé sur le type adéquat de cellules afin de permettre l'expression de sa virulence.

Les virus ayant fait l'objet d'une mesure sont le virus Parvovirus porcin (ci-après noté PPV), le virus Pseudorabies (ci-après noté PRV) impliqué dans la maladie d'Aujeszky, et le virus de l'immunodéficience humaine de type 1 (ci-après noté HIV-1), le virus de l'hépatite A (ci-après noté HAV).

Les mesures de charge virale ont été effectuées par détection de production virale dans des cellules infectées, et plus particulièrement par observation d'un effet spécifique cytopathique. Les virus injectés dans les couches membranaires ont subi soit l'étape de traitement chimique à l'éthanol à 70% v/v selon le procédé, soit l'étape de traitement chimique au peroxyde d'hydrogène à 30% w/v puis 3% w/v selon le procédé.

Chaque mesure de la charge virale a été effectuée deux fois pour chaque essai, celles-ci sont notées dans le tableau suivant par les indications Run A et Run B.

Les résultats dans le tableau suivant représentent la diminution de la charge virale mesurée, exprimée en log. Cette diminution est mesurée par comparaison de la charge virale sur la couche membranaire avant, puis après le traitement indiqué.

| Résultats | | |
|---|---|---|
| **Virus** | **Traitement H$_2$O$_2$** | **Traitement Ethanol** |
| **PPV** | Run A = 4.53 Run B = 3.94 | Run A = 1.43 Run B = 2.03 |
| **PRV** | Run A > 2.89 Run B > 3.13 | Run A > 5.18 Run B > 4.83 |
| **HAV** | Run A > 3.66 Run B > 4.02 | Run A = 1.37 Run B = 1.37 |
| **HIV-1** | Non applicable | Run A > 4.52 Run B > 4.50 |

L'efficacité des traitements chimiques selon le procédé, appliqués aux couches membranaires, et destinés à la viro-inactivation peut être constatée.

Le traitement chimique au peroxyde d'hydrogène est efficace contre le PPV, le PRV et l'HAV. Concernant le HIV-1, les conditions de réalisation des mesures n'ont pas pu donner de résultats interprétables.

Le traitement chimique à l'éthanol est efficace contre le PRV et le HIV-1, et dans une moindre mesure efficace contre les virus nus (non enveloppés) comme le PPV et l'HAV

Comme démontré, seule la combinaison des deux traitements chimiques permet d'assurer une viro-inactivation des couches membranaires, tant des virus enveloppés que des virus nus. Cette viro-inactivation étant définie par une diminution de la population virale d'au moins 4 log.

**Exemple 2 bis : Démonstration de l'efficacité de la viro-inactivation de la couche membranaire selon le procédé.**

Des mesures complémentaires de la charge virale ont été effectuées dans des membranes chorioamniotiques, par injection de virus entre l'amnios et le chorion afin de vérifier la viro-inactivation des tissus selon le procédé.

Des aliquotes des membranes ainsi traitées sont prélevées afin d'être mises en solution et centrifugées. La fraction soluble sert à ensemencer des cellules en milieu de culture cellulaire. Chaque type de virus étudié est ensemencé sur le type adéquat de cellules afin de permettre l'expression de sa virulence.

Les mesures de charge virale ont été effectuées par détection de production virale dans des cellules infectées, et plus particulièrement par observation d'un effet spécifique cytopathique. Les virus ayant fait l'objet d'une mesure sont le virus Pseudorabies (ci-après noté PRV) impliqué dans la maladie d'Aujeszky, et le virus de l'hépatite A (ci-après noté HAV).

Les virus injectés dans les couches membranaires ont subi l'étape de traitement chimique au peroxyde d'hydrogène à 30% w/v puis 3% w/v selon le procédé.

Les résultats dans le tableau suivant représentent la diminution de la charge virale mesurée, exprimée en log. Cette diminution est mesurée par comparaison de la charge virale sur la couche membranaire avant, puis après le traitement indiqué.

| Résultats | |
|---|---|
| **Virus** | **Traitement H$_2$O$_2$** |
| **PRV** | Run A > 4,47 Run B > 4,47 |
| **HAV** | Run A > 4,47 Run B > 4,53 |

L'efficacité des traitements chimiques selon le procédé, appliqués aux couches membranaires, et destinés à la viro-inactivation peut être constatée.

Le traitement chimique au peroxyde d'hydrogène est efficace contre le PRV et l'HAV.

Comme démontré, le traitement au peroxyde d'hydrogène selon le procédé permet d'assurer une viro-inactivation des couches membranaires envers le virus PRV et le virus HAV. Cette viro-inactivation étant définie par une diminution de la population virale d'au moins 4 log.

**Exemple 2 ter : Démonstration de l'efficacité de la viro-inactivation de la couche membranaire selon le procédé.**

Des mesures de la charge virale ont été effectuées dans des membranes amniotiques dont la liaison avec leurs membranes tissulaires spongieuses a été préservée, par injection de virus entre la membrane amniotique et la membrane tissulaire spongieuse afin de vérifier la viro-inactivation des tissus selon le procédé.

Des aliquotes des membranes ainsi traitées sont prélevées afin d'être mises en solution et centrifugées. La fraction soluble sert à ensemencer des cellules en milieu de culture cellulaire. Chaque type de virus étudié est ensemencé sur le type adéquat de cellules afin de permettre l'expression de sa virulence.

Les virus ayant fait l'objet d'une mesure sont le virus Parvovirus porcin (ci-après noté PPV) et le virus de l'immunodéficience humaine de type 1 (ci-après noté HIV-1).

Les mesures de charge virale ont été effectuées par détection de production virale dans des cellules infectées, et plus particulièrement par observation d'un effet spécifique cytopathique. Les virus injectés dans les couches membranaires ont subi l'étape de traitement chimique au peroxyde d'hydrogène à 30% w/v puis 3% w/v selon le procédé.

Chaque mesure de la charge virale a été effectuée deux fois pour chaque essai, celles-ci sont notées dans le tableau suivant par les indications Run A et Run B.

Les résultats dans le tableau suivant représentent la diminution de la charge virale mesurée, exprimée en log. Cette diminution est mesurée par comparaison de la charge virale sur la couche membranaire avant, puis après le traitement indiqué.

| Résultats | |
|---|---|
| **Virus** | **Traitement $H_2O_2$** |
| **HIV-1** | Run A > 6,02<br>Run B > 5,90 |
| **PPV** | Run A = 6,47<br>Run B = 5,66 |

L'efficacité de l'étape de traitement chimique au peroxyde d'hydrogène à 30% w/v puis 3% w/v selon le procédé, appliquée aux couches membranaires, et destinée à la viro-inactivation peut être constatée.

Le traitement chimique au peroxyde d'hydrogène, appliqué à une membrane amniotique dont la liaison avec leurs membranes tissulaires spongieuses a été préservée, est efficace contre le PPV et le HIV-1.

Comme démontré, le traitement au peroxyde d'hydrogène selon le procédé permet d'assurer une viro-inactivation des couches membranaires envers le virus HIV-1 et le virus PPV. Cette viro-inactivation étant définie par une diminution de la population virale d'au moins 4 log.

**Exemple 3 : Démonstration de l'efficacité de la conservation des facteurs de croissance des couches membranaires produites selon le procédé.**

Des mesures de facteurs de croissance par immunodosage ELISA ont été effectuées sur des couches membranaires avant application du procédé et après application du procédé.

Sur des couches membranaires, à savoir des membranes amniotiques, la quantité de facteur de croissance, qui est le TGF (Transforming Growth Factor) de type beta 1, est évaluée.

Avant application du procédé, une quantité de TGF beta 1 de 10 à 20 ng/g de produit testé est mesurée.

Après application du procédé, cette quantité est mesurée entre 1,7 et 13,6 ng/g.

Sur des couches membranaires, à savoir des membranes amniotiques et des membranes choriales, la quantité de facteur de croissance, qui est l'EGF (Epidermal Growth Factor), est évaluée.

Avant application du procédé, une quantité d'EGF d'environ 0,4 à 1,6 ng/g de produit testé est mesurée.

Après application du procédé, cette quantité est mesurée entre 0,01 et 0,2 ng/g.

Les taux de préservation des facteurs de croissance mesurés sont très variables, mais démontrent toujours leur présence. En effet, de nombreux critères expliquant ces fluctuations entrent en jeu, par exemple : selon si la membrane amniotique est localisée au niveau du placenta ou plus éloignée, selon les variations individuelles, ou selon la localisation de l'extraction car c'est principalement au niveau de la couche spongieuse que ces facteurs sont concentrés.

**Exemple 3 bis : Démonstration de l'efficacité de la conservation des facteurs de croissance des couches membranaires produites selon le procédé.**

Des mesures complémentaires de facteurs de croissance par immunodosage ELISA ont été effectuées sur des couches membranaires avant application du procédé et après application du procédé.

Les couches membranaires ayant fait l'objet des mesures complémentaires sont des membranes amniotiques et des membranes chorioamniotiques.

Les facteurs de croissance mesurés sont l'EGF (Epidermal Growth Factor), le TGF (Transforming Growth Factor) de type beta 1 et le FGF (Fibroblast Growth Factor).

Les résultats sont présentés dans le tableau suivant sous forme de moyennes des résultats obtenus.

Le taux de préservation des facteurs de croissance après application du procédé est également indiqué en pourcentages.

|  | Amnios avant traitement en ng/g | Amnios après traitement en ng/g | Taux de préservation | Membrane chorioamniotique avant traitement en ng/g | Membrane chorioamniotique après traitement en ng/g | Taux de préservation |
|---|---|---|---|---|---|---|
| **EGF** | 1,11 | 0,06 | 6 % | 0,6 | 0,007 | 1 % |
| **TGF -$\beta$1** | 30,92 | 3,41 | 11 % | 13,39 | 8,64 | 65 % |
| **FGF** | 3,21 | 0,02 | 1 % | 0,71 | 0,15 | 23 % |

Les taux de préservation des facteurs de croissance mesurés sont très variables, mais démontrent toujours leur présence dans la membrane traitée.

**Exemple 4 : Démonstration de la préservation des protéines structurelles des couches membranaires obtenues selon le procédé.**

Des tests de résistance à la traction des membranes amniotiques et chorioamniotiques ont été effectués selon la méthode décrite par Tanaka dans la référence « Tensile properties of amniotic membrane » (Tanaka *et al.,* 2010) avec un calcul effectué avec la formule mathématique :

$$\text{"}R = Fmax L_{échantillon} \times l_{échantillon}\text{ »,}$$

dans laquelle,

R est la force de rupture de l'échantillon ;
$F_{max}$ est la force maximale exercée par l'appareil de mesure ;
$L_{échantillon}$ est l'aire de l'échantillon ;
$l_{échantillon}$ est la largeur de l'échantillon

Ces essais ont pour objectif de comparer les résistances à la traction des membranes non traitées au recueil et des membranes traitées chimiquement, et lyophilisées selon le procédé, les valeurs obtenues sont rassemblées dans le tableau suivant.

| Traitement | Echantillon | Force de rupture (kPa) | Moyenne | Ecart Type |
|---|---|---|---|---|
| Recueil (non traitée) | Membrane amniotique 1 | 9,5 | 8,2 | 4,0 |
| | | 11,4 | | |
| | | 3,8 | | |
| | Membrane chorioamniotique 1 | 9,9 | 9,1 | 5,1 |
| | | 13,9 | | |
| | | 3,8 | | |
| | Membrane amniotique 2 | 9,4 | 7,1 | 3,2 |
| | | 4,9 | | |
| | Membrane chorioamniotique 2 | 4,8 | 5,8 | 1,5 |
| | | 6,9 | | |
| Après traitement chimique et lyophilisation | Membrane amniotique 3 | 5,1 | 10,3 | 4,7 |
| | | 11,4 | | |
| | | 14,4 | | |
| | Membrane chorioamniotique 4 | 17,9 | 17,9 | / |
| | Membrane chorioamniotique 5 | 5,9 | 5,9 | / |

Ces tests de résistance à la traction démontrent que les fibres de protéines d'intérêt de collagène I, de laminine V et d'élastine sont conservées après le traitement chimique, la lyophilisation et la stérilisation. En effet, les différences de résistance entre les membranes non traitées au recueil et les membranes traitées chimiquement, et lyophilisées selon le procédé ne sont pas significatives.

Ces observations ont été confirmées par des essais immunohistochimiques effectués sur des membranes amniotiques et des membranes chorioamniotiques, afin de visualiser et localiser les protéines d'intérêts que sont le collagène I, la laminine V et l'élastine. Ces essais ont été effectués sur des membranes non traitées au recueil, et sur des membranes après avoir subi toutes les étapes du procédé.

Les résultats ont démontré que les protéines d'intérêts étaient conservées après le traitement.

**Exemple 4 bis : Démonstration de la préservation des protéines structurelles des couches membranaires obtenues selon le procédé.**

Des tests de résistance complémentaires à la traction des membranes amniotiques et chorioamniotiques ont été effectués selon la méthode décrite par Tanaka. Pour cet essai les calculs ont été effectués avec la formule :

$$\text{« } R = \frac{Fmax}{E_{échantillon} \times l_{échantillon}} \quad (Pa) \text{ »}$$

*dans laquelle,*
R est la force de rupture de l'échantillon ;
$F_{max}$ est la force maximale exercée par l'appareil de mesure ;
$E_{échantillon}$ est l'épaisseur de l'échantillon ;
$l_{échantillon}$ est la largeur de l'échantillon.

| Traitement | Echantillon | Force de rupture(Kpa) | Moyenne | Ecart Type |
|---|---|---|---|---|
| Membrane amniotique avant traitement | Echantillon n°1 | 905 | 631 | 274 |
|  |  | 357 |  |  |
|  | Echantillon n°2 | 893 | 679 | 303 |
|  |  | 464 |  |  |
| Membrane chorioamniotique avant traitement | Echantillon n°1 | 217 | 261 | 44 |
|  |  | 305 |  |  |
|  |  |  |  |  |
|  | Echantillon n°2 | 104 | 128 | 33 |
|  |  | 151 |  |  |

| Traitement | Echantillon | Force de rupture (Kpa) | Moyenne | Ecart Type |
|---|---|---|---|---|
| Membrane amniotique traitée selon le procédé | Echantillon n°3 | 390 | 351 | 55 |
|  |  | 312 |  |  |
|  | Echantillon n°4 | 520 | 520 | 0 |
| Membrane chorioamniotique traitée selon le procédé | Echantillon n°5 | 381 | 299 | 71 |
|  |  | 261 |  |  |
|  |  | 254 |  |  |
|  | Echantillon n°1 | 247 | 269 | 22 |
|  |  | 291 |  |  |

Ces tests de résistance à la traction démontrent que les fibres de protéines d'intérêt de collagène I, de laminine V et d'élastine sont conservées après le traitement chimique, la lyophilisation et la stérilisation. En effet, les différences de mesures de résistance entre les membranes non traitées au recueil et les membranes traitées chimiquement, et lyophilisées selon le procédé ne sont pas significatives. La résistance mécanique des membranes est préservée. La grande variabilité des résultats obtenus est due à la nature physiologique des échantillons qui rend très difficile la reproductibilité des essais.

**Revendications**

1.  Procédé de préparation d'un matériau d'allogreffe formant une membrane viro-inactivée, lyophilisée et stérile issue de placenta de mammifère et constituée d'au moins une couche membranaire, ledit procédé comprenant au moins deux étapes de viro-inactivation et au moins une étape de lyophilisation et selon lequel :

    • la première étape de viro-inactivation est effectuée en traitant ladite au moins une couche membranaire avec un premier agent de viro-inactivation choisi dans la famille des alcools ;
    • une deuxième étape de viro-inactivation est effectuée après ladite première étape de viro-inactivation, en traitant ladite au moins une couche membranaire avec un deuxième agent de viro-inactivation choisi dans la famille des peroxydes, **caractérisé en ce que** la deuxième étape de viro-inactivation de ladite au moins une couche membranaire comporte deux sous-étapes de traitement par le deuxième agent de viro-inactivation choisi dans la famille des peroxydes :
    • une première sous-étape de traitement s'effectuant avec une solution de peroxyde à une concentration supérieure à 10% w/v pendant moins de 20 minutes ; et
    • une deuxième sous-étape de traitement s'effectuant avec une solution de peroxyde à une concentration inférieure à 5 % w/v pendant une durée supérieure à 50 minutes.

**2.** Procédé selon la revendication 1, selon lequel la couche membranaire est choisie parmi l'amnios, le chorion, et la membrane chorioamniotique dans laquelle l'amnios et le chorion sont reliés entre eux par des ponts protéiques.

**3.** Procédé selon la revendication 1, selon lequel la couche membranaire est la membrane tissulaire spongieuse.

**4.** Procédé selon l'une quelconque des revendications précédentes, selon lequel le premier agent de viro-inactivation est l'éthanol.

**5.** Procédé selon l'une quelconque des revendications précédentes, selon lequel le deuxième agent de viro-inactivation est le peroxyde d'hydrogène.

**6.** Procédé selon l'une quelconque des revendications précédentes, selon lequel le deuxième agent de viro-inactivation est le peroxyde d'hydrogène sous forme de solution aqueuse dans une concentration comprise entre 3% et 30% w/v.

**7.** Procédé selon l'une quelconque des revendications précédentes, selon lequel la deuxième étape de viro-inactivation de ladite au moins une couche membranaire comporte deux sous-étapes de traitement par le peroxyde d'hydrogène :

• une première sous-étape de traitement s'effectuant avec une solution de peroxyde d'hydrogène à 30% w/v pendant environ 15 minutes ; et
• une deuxième sous-étape de traitement s'effectuant avec une solution de peroxyde d'hydrogène à 3% w/v pendant environ 60 minutes.

**8.** Procédé selon l'une quelconque des revendications précédentes, lequel comporte en outre une étape de lyophilisation de ladite au moins une couche membranaire viro-inactivée.

**9.** Procédé selon l'une quelconque des revendications précédentes, lequel comporte une étape de lyophilisation de ladite au moins une couche membranaire viro-inactivée selon laquelle ladite au moins une couche membranaire viro-inactivée est prise en sandwich entre deux supports sensiblement plats.

**10.** Procédé selon l'une quelconque des revendications précédentes, lequel comporte une étape de lyophilisation de ladite au moins une couche membranaire viro-inactivée, étape selon laquelle ladite au moins une couche membranaire viro-inactivée est soumise à une lyophilisation dans les conditions suivantes :

• une congélation en deux étapes :

- la première étape de congélation s'effectuant à une température d'acclimatation choisie pour ne pas endommager l'intégrité structurelle, fonctionnelle et biologique de la couche membranaire ;
- la deuxième étape de congélation s'effectuant à la température finale de congélation qui est inférieure à la température d'acclimatation ;

• une lyophilisation en deux étapes principales, dites primaire et secondaire :

- l'étape de lyophilisation primaire s'effectuant par application d'un vide à environ 200 microbars et d'un profil de température ascendant ;
- l'étape secondaire de lyophilisation s'effectuant par application d'un vide à environ 50 microbars et d'un profil de température descendant.

**11.** Procédé selon l'une quelconque des revendications précédentes, lequel comporte en outre une étape de stérilisation de la couche membranaire viro-inactivée et lyophilisée, par exposition de cette dernière à des rayonnements gamma à 25-32 kGrays.

**12.** Matériau d'allogreffe viro-inactivé et lyophilisé, susceptible d'être obtenu selon l'une quelconque des revendications de procédé 1 à 11 précédentes, **caractérisé en ce qu'**il est issu de placenta et constitué d'au moins une couche membranaire choisie parmi l'amnios, le chorion, et la membrane chorioamniotique dans laquelle l'amnios et le chorion sont reliés entre eux par des ponts protéiques.

**13.** Matériau d'allogreffe viro-inactivé et lyophilisé selon la revendication 12, présenté sous une forme utilisable comme greffe choisie dans le groupe consistant en la greffe de ligament, la greffe de fascia lata, la greffe de tendon, la greffe de dure-mère, la greffe de membrane sous-muqueuse, la greffe de manchon nerveux, la greffe de coiffe rotateur, la greffe de tissu conjonctival, les agents de traitement de plaies et/ou d'ulcères, et les agents d'amélioration de sutures.

14. Matériau injectable viro-inactivé et lyophilisé, susceptible d'être obtenu selon l'une quelconque des revendications de procédé 3 à 11 précédentes, **caractérisé en ce qu'**il est issu de placenta et constitué d'au moins une couche membranaire qui est la membrane tissulaire spongieuse.

15. Matériau injectable viro-inactivé et lyophilisé, susceptible d'être obtenu selon la revendication 14 se présentant sous une forme adaptée à un traitement par injection choisi dans le groupe consistant en le traitement d'injection intra-articulaire de l'état préarthrosique ou arthrosique et la réparation ligamentaire, méniscale ou cartilagineuse.

**Patentansprüche**

1. Verfahren zur Herstellung eines Allotransplantatmaterials, das eine virusinaktivierte, gefriergetrocknete und sterile Membran bildet, die von Säugetierplazenta stammt und aus mindestens einer Membranschicht besteht, wobei das Verfahren mindestens zwei virusinaktivierende Schritte und mindestens einen Gefriertrocknungsschritt umfasst und gemäß dem:

   - der erste Virusinaktivierungsschritt durch Behandeln der mindestens einen Membranschicht mit einem ersten, aus der Familie der Alkohole ausgewählten Virusinaktivierungsmittel durchgeführt wird;
   - ein zweiter Virusinaktivierungsschritt nach dem ersten Virusinaktivierungsschritt durchgeführt wird, indem die mindestens eine Membranschicht mit einem zweiten virusinaktivierenden Mittel, das aus der Familie der Peroxide ausgewählt ist, behandelt wird,

   **dadurch gekennzeichnet, dass** der zweite Schritt der Virusinaktivierung der mindestens einen Membranschicht zwei Behandlungsteilschritte mit dem aus der Familie der Peroxide ausgewählten, zweiten virusinaktivierenden Mittel umfasst:

   - einen ersten Behandlungsteilschritt mit einer Peroxidlösung mit einer Konzentration von mehr als 10% Gew./Vol. für weniger als 20 Minuten; und
   - einen zweiten Behandlungsteilschritt mit einer Peroxidlösung mit einer Konzentration von weniger als 5 % Gew./Vol. über einen Zeitraum von mehr als 50 Minuten.

2. Verfahren nach Anspruch 1, wobei die Membranschicht ausgewählt ist aus Amnion, Chorion und chorioamniotischer Membran, in der Amnion und Chorion durch Proteinbrücken miteinander verbunden sind.

3. Verfahren nach Anspruch 1, wobei die Membranschicht eine schwammartige Gewebemembran ist.

4. Verfahren nach einem der vorangehenden Ansprüche, wobei das erste virusinaktivierende Mittel Ethanol ist.

5. Verfahren nach einem der vorangehenden Ansprüche, wobei das zweite virusinaktivierende Mittel Wasserstoffperoxid ist.

6. Verfahren nach einem der vorangehenden Ansprüche, wobei das zweite virusinaktivierende Mittel Wasserstoffperoxid in Form einer wässrigen Lösung mit einer Konzentration zwischen 3 % und 30 % Gew./Vol. ist.

7. Verfahren nach einem der vorangehenden Ansprüche, wobei der zweite Schritt der Virusinaktivierung der mindestens einen Membranschicht zwei Behandlungsteilschritte mit Wasserstoffperoxid umfasst:

   - einen ersten Behandlungsteilschritt mit einer Wasserstoffperoxidlösung mit 30 % Gew./Vol. für etwa 15 Minuten; und
   - einen zweiten Behandlungsteilschritt mit einer Wasserstoffperoxidlösung mit 3% Gew./Vol. für etwa 60 Minuten.

8. Verfahren nach einem der vorangehenden Ansprüche, weiter umfassend einen Gefriertrocknungsschritt der mindestens einen virusinaktivierten Membranschicht.

**9.** Verfahren nach einem der vorangehenden Ansprüche, umfassend einen Gefriertrocknungsschritt der mindestens einen virusinaktivierten Membranschicht, gemäß dem die mindestens eine virusinaktivierte Membranschicht zwischen zwei im Wesentlichen flachen Trägern sandwichartig angeordnet wird.

**10.** Verfahren nach einem der vorangehenden Ansprüche, umfassend einen Gefriertrocknungsschritt der mindestens einen virusinaktivierten Membranschicht, wobei gemäß dem Schritt die mindestens eine virusinaktivierte Membranschicht einer Gefriertrocknung unter den folgenden Bedingungen unterzogen wird:

- einem Einfrieren in zwei Schritten, wobei
- der erste Schritt des Einfrierens bei einer Akklimatisierungstemperatur durchgeführt wird, die so gewählt wird, dass die strukturelle, funktionelle und biologische Integrität der Membranschicht nicht beschädigt wird;
- der zweite Schritt des Einfrierens bei der endgültigen Einfriertemperatur durchgeführt wird, die niedriger als die Akklimatisierungstemperatur ist;
- einer Gefriertrocknung in zwei Hauptschritten, als erster und zweiter Hauptschritt bezeichnet; wobei
- der erste Hauptschritt der Gefriertrocknung durchgeführt wird durch Anlegen eines Vakuums mit etwa 200 Mikrobar und einem aufsteigenden Temperaturprofil;
- der zweite Hauptschritt der Gefriertrocknung durchgeführt wird durch Anlegen eines Vakuums mit etwa 50 Mikrobar und einem absteigenden Temperaturprofil.

**11.** Verfahren nach einem der vorangehenden Ansprüche, weiter umfassend eine Sterilisierungsschritt der virusinaktivierten und gefriergetrockneten Membranschicht durch Aussetzen derselben einer Gammastrahlung mit 25-32 kGray.

**12.** Virusinaktiviertes und gefriergetrocknetes Allotransplantat-Material, das nach einem der vorangehenden Verfahrensansprüche 1 bis 11 erhältlich ist, **dadurch gekennzeichnet, dass** das Material von Plazenta stammt und mindestens eine Membranschicht ausbildet, die ausgewählt ist aus Amnion, Chorion und chorioamniotischer Membran, in der Amnion und Chorion durch Proteinbrücken miteinander verbunden sind.

**13.** Virusinaktives und gefriergetrocknetes Allotransplantat-Material nach Anspruch 12, vorliegend in einer als Transplantat geeigneten Form, wobei das Transplantat ausgewählt ist aus der Gruppe bestehend aus Ligamenttransplantat, Fascia-Lata-Transplantat, Sehnentransplantat, Hirnhaut-Transplantat, Submukosamembran-Transplantat, Nervenmanschetten-Transplantat, Rotatorenmanschetten-Transplantat, Bindegewebstransplantat, Wund- und/oder Ulkusbehandlungsmittel und Nahtverbesserungsmittel.

**14.** Virusinaktiviertes und gefriergetrocknetes injizierbares Material, erhältlich nach einem der vorhergehenden Verfahrensansprüche 3 bis 11, **dadurch gekennzeichnet, dass** es aus der Plazenta stammt und eine Membranschicht bildet, die eine schwammartige Gewebemembran ist.

**15.** Virusinaktiviertes und gefriergetrocknetes injizierbares Material, erhältlich nach Anspruch 14, in einer für Behandlung mittels Injektion geeigneten Form, wobei die Behandlung ausgewählt aus der Gruppe bestehend aus intraartikulärer Injektionsbehandlung des präarthritischen oder arthritischen Zustands und der Bänder-, Meniskus- oder Knorpelreparatur.

## Claims

**1.** A method for preparing an allograft material forming a virally inactivated, lyophilized, and sterile membrane obtained from mammalian placenta and consisting of at least one membrane layer, said method including at least two viral inactivation steps and at least one lyophilization step, and according to which:

• the first viral inactivation step is carried out by treating said at least one membrane layer with a first viral inactivation agent selected from the family of the alcohols;
• a second step of viral inactivation is carried out after said first step of viral inactivation, by treating said at least one membrane layer with a second viral inactivation agent selected from the family of the peroxides,

**characterized in that** the second step of viral inactivation of said at least one membrane layer comprises two substeps of treatment with the second viral inactivation agent selected from the family of the peroxides:

• a first substep of treatment being carried out with a peroxide solution at a concentration of more than 10% w/v for less than 20 minutes; and
• a second treatment substep being carried out with a peroxide solution at a concentration of less than 5% w/v for a duration of more than 50 minutes.

2. The method according to claim 1, according to which the membrane layer is selected from the amnios, the chorion, and the chorioamniotic membrane in which the amnios and the chorion are connected to one another by protein bridges.

3. The method according to claim 1, according to which the membrane layer is the spongy tissue membrane.

4. The method according to any one of the preceding claims, according to which the first viral inactivation agent is ethanol.

5. The method according to any one of the preceding claims, according to which the second viral inactivation agent is hydrogen peroxide.

6. The method according to any one of the preceding claims, according to which the second viral inactivation agent is hydrogen peroxide in the form of an aqueous solution at a concentration between 3% and 30% w/v.

7. The method according to any one of the preceding claims, according to which the second step of viral inactivation of said at least one membrane layer comprises two substeps of treatment with hydrogen peroxide:

• a first treatment substep being carried out with a 30% w/v hydrogen peroxide solution for approximately 15 minutes; and
• a second treatment substep being carried out with a 3% w/v hydrogen peroxide solution for approximately 60 minutes.

8. The method according to any one of the preceding claims, which moreover comprises a step of lyophilization of said at least one virally inactivated membrane layer.

9. The method according to any one of the preceding claims, which comprises a step of lyophilization of said at least one virally inactivated membrane layer according to which said at least one virally inactivated membrane layer is sandwiched between two substantially flat supports.

10. The method according to any one of the preceding claims, which comprises a step of lyophilization of said at least one virally inactivated membrane layer, step according to which said at least one virally inactivated membrane layer is subjected to a lyophilization under the following conditions:

• freezing in two steps:

- the first freezing step being carried out at an acclimation temperature selected so as not to damage the structural, functional and biological integrity of the membrane layer;
- the second freezing step being carried out at the final freezing temperature which is below the acclimation temperature;

• a lyophilization in two main steps, referred to as primary step and secondary step:

- the primary lyophilization step being carried out by application of a vacuum at approximately 200 microbar and of a rising temperature profile;
- the secondary lyophilization step being carried out by application of a vacuum at approximately 50 microbar and of a falling temperature profile.

11. The method according to any one of the preceding claims, which moreover comprises a step of sterilization of the virally inactivated and lyophilized membrane layer, by exposing the latter to gamma radiation at 25-32 kGray.

12. Virally inactivated and lyophilized allograft material which can be obtained by any one of the preceding method claims 1 to 11, **characterized in that** it is obtained from placenta and consists of at least one membrane layer selected from the amnios, the chorion, and the chorioamniotic membrane in which the amnios and the chorion are connected to one another by protein bridges.

13. Virally inactivated and lyophilized allograft material according to claim 12, presented in a form which can be used as a graft selected from the group consisting of ligament graft, fascia lata graft, tendon graft, dura mater graft, submucosal membrane graft, nerve sheath graft, rotator cuff graft, conjunctival tissue graft, wound and/or ulcer treatment agents, and suture improvement agents.

14. Virally inactivated and lyophilized injectable material, which can be obtained according to any one of the preceding method claims 3 to 11, **characterized in that** it is obtained from placenta and consists of at least one membrane layer, namely the spongy tissue membrane.

15. Virally inactivated and lyophilized injectable material, which can be obtained according to claim 14, which is in a form suitable for a treatment by injection, selected from the group consisting of the treatment by intra-articular injection of the prearthritic or arthritic condition and ligament, meniscus, or cartilage repair.

**Figure 1**

**Figure 2a**

**Figure 2b**

## RÉFÉRENCES CITÉES DANS LA DESCRIPTION

**Documents brevets cités dans la description**

- US 6152142 A
- US 8709494 B
- CN 104083803
- US 20130247517 A

- US 6024735 A
- US 6482584 B
- FR 2949042

**Littérature non-brevet citée dans la description**

- **NAKAMURA.** *Investigative Ophthalmology & Visual Science,* Janvier 2004, vol. 45 (1
- **T DEPAULA C.A. et al.** Effects of Hydrogen Peroxyde Cleaning Procédures on Bone Graft Osteoinductivity and Mechanical Properties. *Cell Tissue Bank,* 2005, vol. 6 (4), 287-98

- **MBITHI J. N. et al.** *Appl. Environ. Microbiol.,* 1990, vol. 12 (1), 147-179